# EUROPEAN PATENT SPECIFICATION

(11) **EP 3 010 444 B1**
(45) Date of publication and mention of the grant of the patent: **07.06.2023**
(21) Application number: 14741445.2
(22) Date of filing: 16.06.2014
(51) Int. Cl.: A61F 2/16

(54) **HAPTICS FOR INTRAOCULAR DEVICES**
HAPTIK FÜR INTRAOKULARE VORRICHTUNGEN
HAPTIQUES POUR DISPOSITIFS INTRAOCULAIRES

(30) Priority: 16.06.2013 US 201313918969
(43) Date of publication of application: 27.04.2016
(73) Proprietor: VisionCare, Inc., Saratoga, CA (US)
(72) Inventor: AHARONI, Eli, 6940005 Tel Aviv (IL)
(74) Representative: Jaeger, Michael David
(86) International application number: PCT/US2014/042457
(87) International publication number: WO 2014/204826

(56) References cited:
- US-A- 4 409 690
- US-A- 4 579 557
- US-A- 5 814 103
- US-A- 6 152 959
- US-A1- 2013 116 781

## Description

### FIELD OF THE INVENTION

The present invention relates generally to intraocular devices, such as intraocular lenses (IOLs) and particularly to haptics for intraocular devices, such as an IOL.

### BACKGROUND OF THE INVENTION

Haptics are clearly defined in the art as the interface elements of an intraocular device that touch the eye structure ("haptic" is from the Greek word for touch). Haptics are typically outwardly extending (e.g., radially, tangentially, or the like) structures that are attached to the optic and support the optic within the eye by pressing against adjacent tissue.

Both resilient and rigid haptics are known. Resilient haptics are generally resilient wires made of plastics or any other biologically inert material, but which are sufficiently stiff so that when a compressive force is applied thereto, they distort but do not buckle or collapse. Rigid haptics do not deform significantly under compressive or tensile forces; they may be made of significantly stiffer materials than resilient haptics or may be more rigid due to their shape and construction, such as plate haptics.

Although haptics are mostly used in the capsular bag, haptics are also used to affix the device to ocular structure outside the bag. For example, haptics have been inserted in sclerotomies, which are incisions made in the sclera. Currently known fixation methods include bonding the haptics to the ocular structure with an adhesive; making a flap (rectangular, square, triangular, Y-shape or other shapes), inserting the haptic and covering it with the flap, and suturing; or inserting and fixing a haptic in a scleral tunnel. However, these methods have disadvantages. For example, it is difficult to insert haptic loops into sclerotomies. Tilting and decentration may occur due to poor fixation. The haptic loops may become distorted due to manipulation by tools, such as intraocular forceps.

US patent publication No. 6152959 relates to an iris fixated intraocular lens for implanting in the anterior chamber of an eye which includes an optic having an optical axis and anterior and posterior sides, and first and second fixation members, each of the fixation members having a proximal end region and a distal end region. The proximal end region of each fixation member is a single flexible strand fixed to an edge region of the optic to extend generally tangentially outwardly therefrom and the distal end region is formed into a loop having defined therein at least one narrow iris pincher gap for detachably attaching the intraocular lens to the anterior surface of the iris. The first and second fixation members are substantially identical to one another and are attached to the optic on opposite sides of the optical axis.

### SUMMARY OF THE INVENTION

The present invention seeks to provide improved haptics for intraocular devices, as is described further in detail hereinbelow.

There is thus provided in accordance with an embodiment of the present invention an assembly including an intraocular device having anterior and posterior faces, haptics extending from the intraocular device, each of the haptics including a slender loop element with a proximal end attached to the intraocular device, the slender loop element having a length, width and thickness, the length following a curved path and the width being generally parallel to a central anterior-posterior axis of the intraocular device, and a distal hook portion extending from a distal end of the slender loop element, the distal hook portion comprising a surface that extends between an anterior edge and a posterior edge, the distal hook portion is tilted with respect to the width and to the thickness of the slender loop element towards the central anterior-posterior axis of the intraocular device so that said anterior edge is radially more proximal than said posterior edge with respect to said central anterior posterior axis.

Non-limiting features of the invention include the following, among others:
The distal hook portion is round at its distal tip and tapers to be thinner from where it extends from the slender loop element.

The distal hook portion is thinner than the slender loop element.

The thickness of the slender loop elements is less than the width.

The distal hook portion is formed with a through hole.

The proximal end of the slender loop element is generally tangential to an outer periphery of the intraocular device.

The distal hook portion and the slender loop element can have different rigidity; the slender loop element and the intraocular device can have different rigidity.

The anterior-most surface of the distal hook portion is not posterior to the anterior-most surface of the intraocular device; the anterior-most surface of the slender loop element is not posterior to the anterior-most surface of the intraocular device.

In accordance with an embodiment of the present invention the intraocular device includes an IOL, a scleral fixation bag or an implantable miniature telescope.

In accordance with an embodiment of the present invention, the flat and angled loop edges of the haptics help fixate and stabilize the lens or other ocular device. The flat loop profile is shaped for tangential placement in the sclera (i.e., tangent to the sclera), providing better stabilization and preventing tilting and decentration of the lens or other intraocular device. The distal hook portion eases the manipulation of the assembly/device in the eye. The hooked loops at the distal haptic ends significantly facilitate implantation directly into sclerotomies.

### BRIEF DESCRIPTION OF THE DRAWINGS

The present invention will be understood and appreciated more fully from the following detailed description taken in conjunction with the appended drawings in which:
Fig. 1 is a simplified pictorial illustration of an intraocular assembly (IOL) with haptics, constructed and operative in accordance with an embodiment of the present invention;
Fig. 2A is a simplified pictorial illustration of the intraocular assembly of Fig. 1 implanted in an eye, viewed along the anterior-posterior axis, wherein the haptics are fixed in a curved sclerotomy, in accordance with an embodiment of the present invention;
Fig. 2B is a simplified illustration of the haptics in the sclerotomy, viewed transverse to the anterior-posterior axis;
Fig. 3 is a simplified pictorial illustration of an intraocular assembly (implantable miniature telescope) with haptics, constructed and operative in accordance with an embodiment of the present invention; and
Fig. 4 is a simplified pictorial illustration of an IOL with haptics.

### DETAILED DESCRIPTION OF THE PRESENT INVENTION

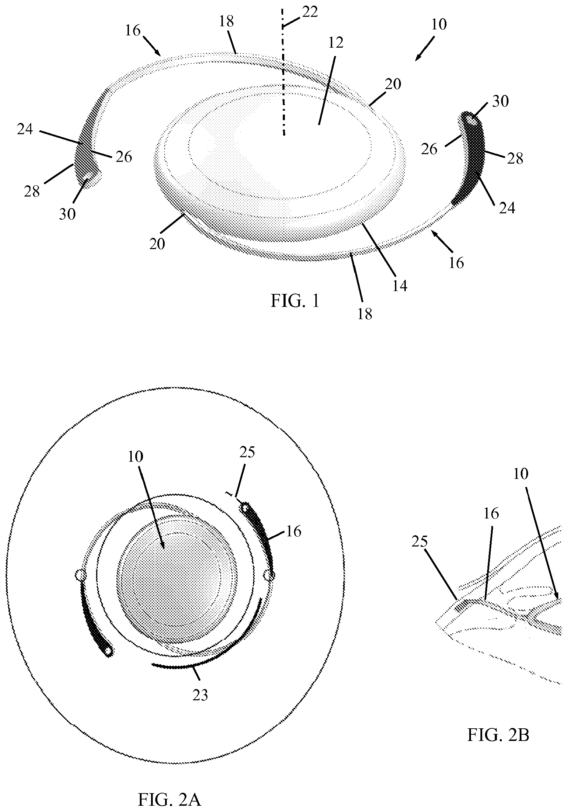

Reference is now made to Figs. 1-2B, which illustrate an intraocular assembly, constructed and operative in accordance with an embodiment of the present invention.

The assembly includes an intraocular device 10 having anterior and posterior faces 12 and 14. In this embodiment, intraocular device 10 is an IOL (such as, but not limited to, a monofocal IOL, multifocal IOL, accommodating IOL and others). Haptics 16 extend from intraocular device 10. Each haptic 16 includes a slender loop element 18 with a proximal end 20 attached to intraocular device 10. Slender loop element 18 has a length, width and thickness; its length follows a curved path and its width is parallel to a central anterior-posterior axis 22 of intraocular device 10. The outer diameter of intraocular device 10 with haptics 16 is, without limitation, 15 mm for proper mounting in the sclera.

A distal hook portion 24 extends from a distal end of slender loop element 18. Distal hook portion 24 includes an anterior edge 26 tilted towards the central anterior-posterior axis 22 of intraocular device 10. Preferably, but not necessarily, distal hook portion 24 is flat. The shape of distal hook portion 24 may match the shape of its insertion place in the sclera. Distal hook portion 24 is rounded at its distal tip and tapers to be thinner from where it extends from slender loop element 18. Distal hook portion 24 may be formed with a through hole 30, for grasping or dialing or other adjustments. Through hole 30 is preferably central and not shifted to one side. In one embodiment, distal hook portion 24 is thinner than slender loop element 18. In one embodiment, the thickness of slender loop elements 18 is less than its width; in other embodiments they are equal.

The proximal end of slender loop element 18 is generally tangential to the outer periphery of intraocular device 10.

The lens portion or optic, as well as the other portions of the assembly including the haptics, may be made from one or more materials that are biocompatible and optically transparent, and can be hydrophilic or hydrophobic. The material may be rigid or flexible, hard or soft, such as without limitation, methacrylates (e.g., polymethyl methacrylate), olefins (e.g., polypropylene), and silicones.

The distal hook portion 24 and the slender loop element 18 can have different rigidity. Similarly, slender loop element 18 and intraocular device 10 can have different rigidity.

Reference is now made to Figs. 2A and 2B. The intraocular device 10 is implanted, such as through an incision 23 in the limbus. The haptics 16 are fixed in curved sclerotomies 25 and properly center the device 10.

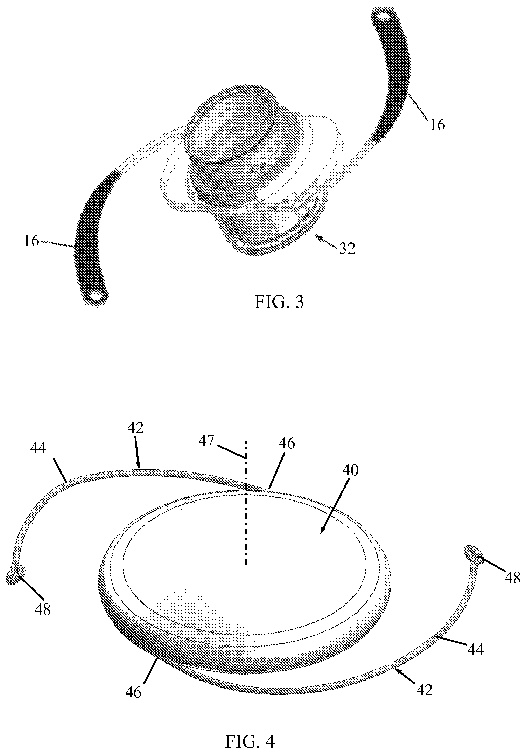

Reference is now made to Fig. 3, in which the intraocular assembly is an implantable miniature telescope 32 with haptics 16, constructed and operative in accordance with an embodiment of the present invention. The haptics 16 are as described above. The haptics can be used with other intraocular devices, such as a scleral fixation bag, which is described in a copending application.

Reference is now made to Fig. 4, which illustrates an IOL 40 with haptics 42, constructed and operative in accordance with another arrangement. As in the embodiments of the invention, each haptic 42 includes a slender loop element 44 with a proximal end 46 attached to IOL 40 and a distal hook portion 48 whose anterior edge is tilted towards the central anterior-posterior axis 47 of IOL 40. In this embodiment, slender loop element 44 is a slender wire and distal hook portion 48 is a rounded eyelet bent or otherwise formed from the end of the slender wire.

## Claims

1. An assembly comprising:
an intraocular device (10) having anterior and posterior faces (12, 14); and
haptics (16) extending from said intraocular device (10), each of said haptics (16) comprising a slender loop element (18) with a proximal end attached to said intraocular device (10), said slender loop element (18) having a length, width and thickness, the length following a curved path and the width being generally parallel to a central anterior-posterior axis (22) of said intraocular device (10),
**characterised by** a distal hook portion (24) extending from a distal end of said slender loop element (18), said distal hook portion (24) comprising a surface that extends between an anterior edge (26) and a posterior edge (28), the distal hook portion (24) being tilted with respect to the width and to the thickness of said slender loop element (18) towards the central anterior-posterior axis (22) of said intraocular device (10) so that said anterior edge (26) is radially more proximal than said posterior edge (28) with respect to said central anterior-posterior axis (22).

2. The assembly according to claim 1, wherein said distal hook portion (24) is rounded at its distal tip and tapers to be thinner from where it extends from said slender loop element (18).

3. The assembly according to claim 1, wherein said distal hook portion (24) is thinner than said slender loop element (18).

4. The assembly according to claim 1, wherein the thickness of said slender loop element (18) is less than the width.

5. The assembly according to claim 1, wherein said distal hook portion (24) is formed with a central through hole (30).

6. The assembly according to claim 1, wherein the proximal end of said slender loop element (18) is generally tangential to an outer periphery of said intraocular device (10).

7. The assembly according to claim 1, wherein said distal hook portion (24) and said slender loop element (18) have different rigidity.

8. The assembly according to claim 1, wherein said slender loop element (18) and said intraocular device (10) have different rigidity.

9. The assembly according to claim 1, wherein an anterior-most surface of said distal hook portion (24) is not posterior to an anterior-most surface of said intraocular device (10).

10. The assembly according to claim 1, wherein an anterior-most surface of said slender loop element (18) is not posterior to an anterior-most surface of said intraocular device (10).

11. The assembly according to claim 1, wherein said intraocular device (10) comprises an IOL.

12. The assembly according to claim 1, wherein said intraocular device (10) comprises an implantable miniature telescope (32).

## Patentansprüche

1. Anordnung, umfassend:
eine intraokulare Vorrichtung (10), die eine vordere und eine hintere Fläche (12, 14) aufweist, und
Haptiken (16), die sich von der intraokularen Vorrichtung (10) erstrecken, wobei jede der Haptiken (16) ein schmales Schleifenelement (18) mit einem nahen Ende umfasst, das an der intraokularen Vorrichtung (10) angebracht ist, wobei das schmale Schleifenelement (18) eine Länge, Breite und Dicke aufweist, wobei die Länge einem gekrümmten Weg folgt und die Breite im Allgemeinen parallel zu einer zentralen Vorne-Hinten-Achse (22) der intraokularen Vorrichtung (10) verläuft,
**gekennzeichnet durch** einen fernen Hakenabschnitt (24), der sich von einem fernen Ende des schmalen Schleifenelements (18) erstreckt, wobei der ferne Hakenabschnitt (24) eine Fläche umfasst, die sich zwischen einer Vorderkante (26) und einer Hinterkante (28) erstreckt, wobei der ferne Hakenabschnitt (24) in Bezug auf die Breite und die Dicke des schmalen Schleifenelements (18) hin zur zentralen Vorne-Hinten-Achse (22) der intraokularen Vorrichtung (10) derart geneigt ist, dass sich die Vorderkante (26) radial näher als die Hinterkante (28) in Bezug auf die Vorne-Hinten-Achse (22) befindet.

2. Anordnung nach Anspruch 1, wobei der ferne Hakenabschnitt (24) an seiner fernen Spitze abgerundet ist und sich so verjüngt, dass er dünner ist von dort, wo er sich von dem schmalen Schleifenelement (18) erstreckt.

3. Anordnung nach Anspruch 1, wobei der ferne Hakenabschnitt (24) dünner als das schmale Schleifenelement (18) ist.

4. Anordnung nach Anspruch 1, wobei die Dicke des schmalen Schleifenelements (18) geringer als die Breite ist.

5. Anordnung nach Anspruch 1, wobei der ferne Hakenabschnitt (24) mit einem zentralen Durchgangsloch (30) ausgebildet ist.

6. Anordnung nach Anspruch 1, wobei das nahe Ende des schmalen Schleifenelements (18) allgemein tangential zu einem Außenumfang der intraokularen Vorrichtung (10) ist.

7. Anordnung nach Anspruch 1, wobei der ferne Hakenabschnitt (24) und das schmale Schleifenelement (18) unterschiedliche Steifigkeit aufweisen.

8. Anordnung nach Anspruch 1, wobei das schmale Schleifenelement (18) und die intraokulare Vorrichtung (10) unterschiedliche Steifigkeit aufweisen.

9. Anordnung nach Anspruch 1, wobei eine vorderste Fläche des distalen Hakenabschnitts (24) nicht hinter einer vordersten Fläche der intraokularen Vorrichtung (10) liegt.

10. Anordnung nach Anspruch 1, wobei eine vorderste Fläche des schmalen Schleifenelements (18) nicht hinter einer vordersten Fläche der intraokularen Vorrichtung (10) liegt.

11. Anordnung nach Anspruch 1, wobei die intraokulare Vorrichtung (10) eine IOL umfasst.

12. Anordnung nach Anspruch 1, wobei die intraokulare Vorrichtung (10) ein implantierbares Miniaturteleskop (32) umfasst.

## Revendications

1. Un ensemble comprenant :
un dispositif intraoculaire (10) ayant des faces antérieure et postérieure (12, 14); et
des haptiques (16) s'étendant depuis ledit dispositif intraoculaire (10), chacun desdits haptiques (16) comprenant un élément en boucle mince (18) avec une extrémité proximale fixée audit dispositif intraoculaire (10), ledit élément en boucle mince (18) ayant un longueur, une largeur et un épaisseur, la longueur suivant une trajectoire incurvée et la largeur étant généralement parallèle à un axe central antéropostérieur (22) dudit dispositif intraoculaire (10),
**caractérisé par** une partie de crochet distale (24) s'étendant à partir d'une extrémité distale dudit élément en boucle mince (18), ladite partie de crochet distale (24) comprenant une surface qui s'étend entre un bord antérieur (26) et un bord postérieur (28), la partie de crochet distale (24) étant inclinée par rapport à la largeur et à l'épaisseur dudit élément en boucle mince (18) vers l'axe central antéropostérieur (22) dudit dispositif intraoculaire (10) de sorte que ledit bord antérieur (26) soit radialement plus proximal que ledit bord postérieur (28) par rapport audit axe central antéropostérieur (22).

2. Ensemble selon la revendication 1, dans lequel ladite partie de crochet distale (24) est arrondie à son extrémité distale et se rétrécit pour être plus fine à partir de l'endroit où elle s'étend depuis ledit élément en boucle mince (18).

3. Ensemble selon la revendication 1, dans lequel ladite partie de crochet distale (24) est plus mince que ledit élément en boucle mince (18).

4. Ensemble selon la revendication 1, dans lequel l'épaisseur dudit élément en boucle mince (18) est inférieur à la largeur.

5. Ensemble selon la revendication 1, dans lequel ladite partie de crochet distale (24) est formée avec un trou traversant central (30).

6. Ensemble selon la revendication 1, dans lequel l'extrémité proximale dudit élément en boucle mince (18) est généralement tangentielle à une périphérie externe dudit dispositif intraoculaire (10).

7. Ensemble selon la revendication 1, dans lequel ladite partie de crochet distale (24) et ledit élément en boucle mince (18) ont une rigidité différente.

8. Ensemble selon la revendication 1, dans lequel ledit élément en boucle mince (18) et ledit dispositif intraoculaire (10) ont une rigidité différente.

9. Ensemble selon la revendication 1, dans lequel une surface la plus antérieure de ladite partie distale la partie de crochet (24) n'est pas postérieure à une surface la plus antérieure dudit dispositif intraoculaire (10).

10. Ensemble selon la revendication 1, dans lequel une surface la plus antérieure dudit élément en boucle mince (18) n'est pas postérieure à une surface la plus antérieure dudit dispositif intraoculaire (10).

11. Ensemble selon la revendication 1, dans lequel ledit dispositif intraoculaire (10) comprend une lentille intraoculaire (IOL).

12. Ensemble selon la revendication 1, dans lequel ledit dispositif intraoculaire (10) comprend un télescope miniature implantable (32).
